(19) Europäisches Patentamt
European Patent Office
Office européen des brevets

(11) Publication number: 0 066 373 B1

(12) EUROPEAN PATENT SPECIFICATION

(45) Date of publication of patent specification: 20.03.85

(21) Application number: 82302274.4

(22) Date of filing: 05.05.82

(51) Int. Cl.[4]: C 07 D 499/00, A 61 K 31/43 // C07C143/12, C07C69/157, C07C69/96 ,(A61K31/43, 31/42)

# (54) Beta-lactam derivatives, a process for their preparation and compositions containing them.

(30) Priority: 22.05.81 GB 8115741

(43) Date of publication of application: 08.12.82 Bulletin 82/49

(45) Publication of the grant of the patent: 20.03.85 Bulletin 85/12

(84) Designated Contracting States: BE CH DE FR GB IT LI NL SE

(56) References cited:
EP-A-0 015 690
GB-A-1 530 861

(73) Proprietor: BEECHAM GROUP PLC
Beecham House Great West Road
Brentford Middlesex TW8 9BD (GB)

(72) Inventor: Kenyan, Robert Fletcher
12 Christchurch Gardens Christchurch Mount
Epsom Surrey (GB)
Inventor: Burton, George
14 Windborough Road
Carshalton Surrey (GB)

(74) Representative: Hesketh, Alan, Dr. et al
European Patent Attorney Beecham Pharmaceuticals Great Burgh Yew Tree Bottom Road
Epsom, Surrey, KT18 5XQ (GB)

**The file contains technical information submitted after the application was filed and not included in this specification**

EP 0 066 373 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a class of β-lactam derivatives which have antibacterial activity and are of value in the treatment of infections in animals, including mammals and in particular humans, caused by a wide range of organisms, particularly Gram-negative organisms. In particular the invention relates to a small class of 6α-methoxy penicillin derivatives. The invention also relates to a process for the preparation of such compounds, and to pharmaceutical compositions comprising them.

European Patent Publication No. 0015690 discloses *inter alia* a class of 6-methoxy acylamino penicillins of formula (A) or a pharmaceutically acceptable salt or *in vivo* hydrolysable ester thereof:

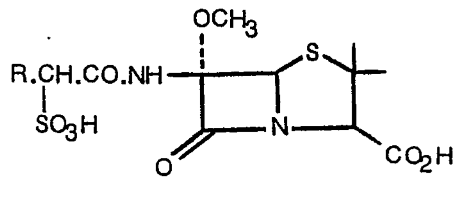

(A)

wherein R is $C_{1-6}$ alkyl; an optionally substituted 5-membered heterocyclic ring containing one or two hetero-atoms selected from oxygen, sulphur and nitrogen; phenyl; mono-substituted phenyl where the substituent is halogen, hydroxy, $C_{1-6}$ alkoxy, nitro, amino, $C_{1-6}$ alkyl, $C_{1-6}$ haloalkyl, $C_{1-6}$ alkylcarbonyloxy, or $C_{1-6}$ alkyl sulphonylamino (for example —$NHSO_2CH_3$); or di-substituted phenyl where the substituents are selected from hydroxy, halogen, methoxy, acetoxy and amino.

We have now found a further small class of novel penicillin derivatives within the scope of compounds disclosed in the above identified European patent application which have a particularly high level of antibacterial activity.

According to the present invention there is provided a compound of formula (I) or a pharmaceutically acceptable salt or *in vivo* hydrolysable ester thereof:

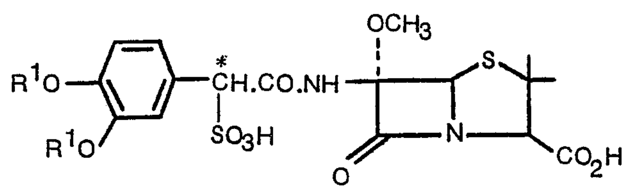

(I)

wherein $R^1$ is hydrogen or acetyl.

The carbon atom marked * in formula (I) is asymmetric so that the compounds may exist as two optically active diasteroiosmers; that containing the R-side chain is the preferred isomer.

The compounds of the present invention include the pharmaceutically acceptable esters of compound (I) which hydrolyse readily in the human body to produce the parent acid, for example acyloxyalkyl groups such as acetoxymethyl, pivaloyloxymethyl, α-acetoxyethyl, α-acetoxybenzyl and α-pivaloyloxyethyl groups; alkoxycarbonyloxyalkyl groups, such as ethoxycarbonyloxymethyl and α-ethoxycarbonyloxyethyl; and lactone groups, such as phthalidyl or dimethoxyphthalidyl.

Suitable salts of the compound of formula (I) include salts of the 3-carboxylic acid group and also of the sulpho groups. Salts include metal salts, e.g. aluminium, alkali metal salts such as sodium or potassium, alkaline earth metal salts such as calcium or magnesium, and ammonium or substituted ammonium salts, for example those with lower alkylamines such as triethylamine, hydroxy-lower alkylamines such as 2-hydroxy-ethylamine, bis-(2-hydroxyethyl)-amine or tri-(2-hydroxyethyl)-amine, cycloalkylamines such as bicyclohexylamine, or with procaine, dibenzylamine, N,N-dibenzylethylenediamine, l-ephenamine, N-ethylpiperidine, N-benzyl-β-phenethylamine, dehydroabietylamine, N,N'-bisdehydroabietylethylenediamine, or bases of the pyridine type such as pyridine, collidine or quinoline, or other amines which have been used to form salts whith known penicillins.

The compounds of formula (I) may be prepared by reacting a compound of formula (II):

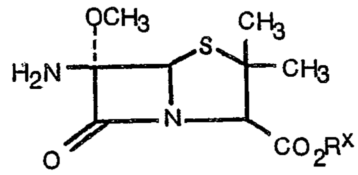

(II)

wherein the amino group is optionally substituted with a group which permits acylation to take place, and wherein $R^x$ is hydrogen or a carboxyl-blocking group, with an N-acylating derivative of an acid of formula (III):

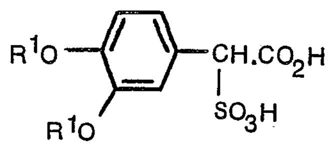

(III)

wherein $R^1$ is defined with respect to formula (I) above; and thereafter, if necessary, carrying out one or more of the following steps:
i) removing any carboxyl-blocking group $R^X$;
ii) removing any substituent on the amide group;
iii) converting the product into a salt or *in vivo* hydrolysable ester thereof.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-*n*-butyltin, groups of formula —P.$R^aR^b$ wherein $R^a$ is an alkyl, haloalkyl, aryl, aralkyl, alkoxy, haloalkoxy, aryloxy, aralkyloxy or dialkylamino group, $R^b$ is the same as $R^a$ or is halogen or $R^a$ and $R^b$ together form a ring; suitable such phosphorus groups being —$P(OC_2H_5)_2$, —$P(C_2H_5)_2$,

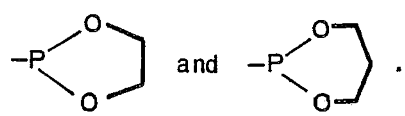

Suitable carboxyl-blocking derivatives for the group —$CO_2R^x$ in formula (II) include salts, ester, and anhydride derivatives of the carboxylic acid. The derivative is preferably one which may readily be cleaved at a later stage of the reaction. Suitable salts include tertiary amine salts, such as those with tri-lower-alkylamines, N-ethylpiperidine, 2,6-lutidine, pyridine, N-methylpyrrolidine, dimethylpiperazine. A preferred salt is with triethylamine.

Suitable ester-forming carboxyl-blocking groups are those which may be removed under conventional conditions. Such groups for $R^x$ include benzyl, p-methoxybenzyl, 2,4,6-trimethylbenzyl, 3,5-di-t-butyl-4-hydroxybenzyl, benzoylmethyl, p-nitrobenzyl, 4-pyridylmethyl, 2,2,2-trichloroethyl, 2,2,2-tribromoethyl, t-butyl, t-amyl, diphenylmethyl, triphenylmethyl, adamantyl, 2-benzyloxyphenyl, 4-methylthiophenyl, tetra-hydrofur-2-yl, tetrahydropyran-2-yl, pentachlorophenyl, p-toluenesulphonylethyl, methoxymethyl, a silyl, stannyl or phosphorus-containing group, such as described above, an oxime radical of formula —N=CHR° where R° is aryl or heterocyclic, or an *in vivo* hydrolysable ester radical such as defined above.

The carboxyl group may be regenerated from any of the above esters by usual methods appropriate to the particular $R^x$ group, for example, acid—and base—catalysed hydrolysis, or by enzymically—catalysed hydroylsis, or by hydrogenation.

A reactive N-acylating derivative of the acid (III) is employed in the above process. The choice of reactive derivative will of course be influenced by the chemical nature of the substituents of the acid.

Suitable N-acylating derivatives include an acid halide, preferably the acid chloride or bromide. Acylation with an acid halide may be affected in the presence of an acid binding agent for example tertiary amine (such as triethylamine or dimethylanaline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide—such as ethylene oxide or propylene oxide. The acylation reaction using an acid halide may be carried out at a temperature in the range −50°C to +50°C, preferably −20°C to +20°C, in aqueous or non-aqueous media such as aqueous acetone, ethyl acetate, dimethylacetamide, dimethylformamide, acetonitrile, dichloromethane, 1,2-dichloroethane, or mixtures thereof. Alternatively,

the reaction may be carried out in an unstable emulsion of water-immiscible solvent, especially an aliphatic ester or ketone, such as methyl isobutyl ketone or butyl acetate.

The acid halide may be prepared by reacting the acid (III) or a salt thereof with a halogenating (e.g. chlorinating or brominating) agent such as phosphorus pentachloride, thionyl chloride or oxalyl chloride.

The acid halide may also be prepared by reacting 2(3,4-diacetoxyphenyl)acetyl chloride or other suitably protected 2(3,4-dihydroxyphenyl)acetyl chloride with a sulphonating agent, such as, for example, the sulphur trioxide-dioxane complex.

Alternatively, the N-acylating derivative of the acid (III) may be a symmetrical or mixed anhydride. Suitable mixed anhydrides are alkoxyformic anhydrides, or anhydrides with, for example carbonic acid monoesters, trimethyl acetic acid, thioacetic acid, diphenylacetic acid, benzoic acid, phosphorus acids (such as phosphoric or phosphorous acids), sulphuric acid or aliphatic or aromatic sulphonic acids (such as *p*-toluenesulphonic acid). The mixed or symmetrical anhydrides may be generated using N-ethoxycarbonyl-2-ethoxy-1,2-dihydroquinoline. When a symmetrical anhydride is employed, the reaction may be carried out in the presence of 2,4-lutidine as catalyst.

Alternative N-acylating derivatives of acid (III) are the acid azide, or activated esters such as esters with 2-mercaptopyridine, cyanomethanol, *p*-nitrophenol, 2,4-dinitrophenol, thiophenol, halophenols, including pentachlorophenol, monomethoxyphenol or 8-hydroxyquinoline; or amides such as N-acylsaccharins or N-acylphthalimides; or an alkylidene iminoester prepared by reaction of the acid (III) with an oxime.

Other reactive N-acylating derivatives of the acid (III) include the reactive intermediate formed by reaction *in situ* with a condensing agent such as a carbodiimide, for example N,N-diethyl-, dipropyl- or diisopropylcarbodiimide, N,N'-di-cyclohexylcarbodiimide, or N-ethyl-N'-γ-dimethylaminopropylcarbodi-imide; a suitable carbonyl compound, for example N,N'-carbonyldiimidazole or N,N'-carbonylditriazole; an isoxasolinium salt, for example N-ethyl-5-phenylisoxazolinium-3-sulphonate or N-*t*-butyl-5-methylisoxazolinium perchlorate; or an N-alkoxycarbonyl-2-alkoxy-1,2-dihydroquinoline, such as N-ethoxycarbonyl-2-ethoxy-1,2-dihydroquinoline. Other condensing agents include Lewis acids (for example $BBr_3—C_6H_6$); or a phosphoric acid condensing agent such as diethylphosphorylcyanide. The condensation reaction is preferably carried out in an organic reaction medium, for example methylene chloride, dimethylformamide, acetonitrile, alcohol, benzene, dioxan, or tetrahydrofuran.

The starting material of formula (II) is disclosed in British Patent No. 1,339,007.

Compounds of formula (I) may also be prepared by reacting a compound of formula (IV):

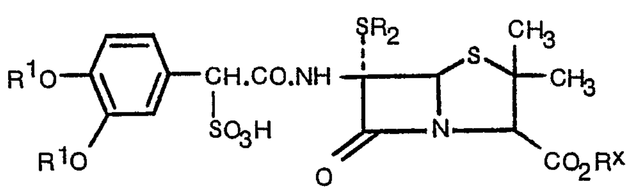

(IV)

wherein $R^1$ and $R^x$ are as defined above and $R^2$ is $C_1$ to $C_6$ alkyl, aryl or benzyl; with methanol in the presence of a metal ion, such as mercury, lead, silver, cadmium, thallium, tellurium or bismuth.

This reaction may generally be carried out at a temperature of from −50°C to +25°C, but is conveniently carried out between −5°C and +25°C. Any suitable solvent may be employed. It is generally convenient however to use methanol as the solvent. Preferably $R^2$ in formula (IV) represents methyl.

Compounds of formula (I) may also be prepared by:

a) treating a compound of formula (V):

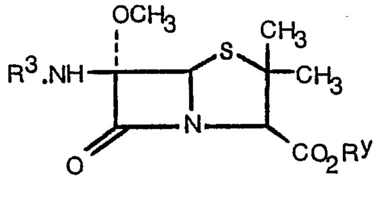

(V)

wherein $R^y$ is a carboxyl-blocking group, and $R^3$ is an acyl group, in particular an acyl group derived from the side-chain of a natural penicillin, such as benzyl penicillin or phenoxymethyl penicillin; with an agent forming an amino halide;

b) treating the imino halide with a compound to introduce a group $QR_f$ on the imino carbon atom, wherein Q is oxygen, sulphur or nitrogen and $R_f$ is an alkyl group of from 5 to 14 carbon atoms, to form an iminoether, iminothioether, or amidine (when Q is O, S, or N respectively);

c) Reacting with an N-acylating derivative of an acid of formula (III) above.
d) treating with water; and
e) optionally removing the carboxyl-blocking group $R^y$.

A suitable agent for preparing an amino halide is an acid halide in the presence of an acid binding agent such as a tertiary amine, e.g. pyridine, triethylamine, or N,N-dimethylaniline. Examples of suitable acid halides are phosphorus pentachloride, phosgene, phosphorous pentabromide, phosphorus oxychloride, oxalyl chloride and p-toluene sulphonic acid chloride. Phosphorus pentachloride and phosphorus oxychloride are preferred. The reaction may be conducted under cooling, preferably at temperatures from 0°C to −30°C when phosphorus pentachloride is employed. The amount of the tertiary amine is preferably 3—5 mols per mol of phosphorus pentachloride. It is also preferable to use the phosphorus halide in an amount slightly in excess of that of the starting material.

The resulting imino compounds are then treated to introduce a —$OR_f$ group onto the imino carbon atom. This is preferably effected by reacting the imino halide with a corresponding alcohol. Examples of suitable alcohols for reaction with the imino halide are aliphatic alcohols containing from 1 to 12 carbon atoms, preferably 1 to 5 carbon atoms, such as methanol, ethanol, propanol, isopropyl alcohol, amyl alcohol and butyl alcohol, and aralkyl alcohols such as benzyl alcohol and 2-phenylethanol.

The reaction of the alcohol with the imino halide is preferably effected in the presence of an acid binding agent, such as a tertiary amine, preferably pyridine, and the reaction is usually carried out without isolating the imino halide from the reaction mixture.

Finally, the product is treated with water. The water treatment may be conducted together with the isolation of the desired material. That is the reaction mixture may be added to water or a saturated aqueous solution of sodium chloride and then the aqueous layer formed is separated from the organic solvent layer.

The antibiotic compounds according to the invention may be formulated for administration in any convenient way for use in human or veterinary medicine, by analogy with other antibiotics, and the invention therefore includes within its scope a pharmaceutical composition comprising a compound of formula (I) above together with a pharmaceutical carrier or excipient.

The compositions may be formulated for administration by any route, such as oral topical or parenteral. The compositions may be in the form of tablets, capsules, powders, granules, lozenges, creams of liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maizestarch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl *p*-hydroxybenzoate or sorbic acid, and if desired conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilized before filling into a suitable vial or ampoule and sealing. Advantageously, agents such as a local anesthetic, preservative and buffering agents can be dissolved in the vehicle. To enhance the stability, the composition can be frozen after filling into the vial and the water removed under vaccuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

The compositions may contain from 0.1% by weight, preferably from 10—60% by weight, of the active material, depending on the method of administration. Where the compositions comprise dosage units, each unit will preferably contain from 50—500 mg. of the active ingredient. The dosage as employed for adult human treatment will preferably range from 100 to 3000 mg. per day, for instance 1500 mg. per day depending on the route and frequency of administration.

The compound of formula (I) may be the sole therapeutic agent in the compositions of the invention or a combination with other antibiotics and/or β-lactamase inhibitors may be employed.

Advantageously the compositions also comprise a compound of formula (VI) or a pharmaceutically acceptable salt or ester thereof:

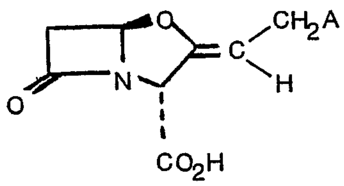

(VI)

wherein A is hydroxyl, substituted hydroxyl, thiol, substituted thiol, amino, mono- or di-hydrocarbyl-substituted amino, or mono- or di-acylamino.

A further advantageous composition comprises a compound of formula (I) or a pharmaceutically acceptable salt or *in vivo* hydrolysable ester thereof together with a compound of formula (VII) or a pharmaceutically acceptable salt or *in vivo* hydrolysable ester thereof:

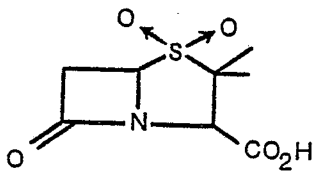

(VII)

The following Examples illustrate the preparation of some of the compounds of this invention.

PREPARATION 1

2-(3,4-Diacetoxyphenyl)acetic acid

2-(3,4-Dihydroxyphenyl)acetic acid (4.70 g, 23.3 mmole) in acetic anhydride (100 ml) and pyridine (1 ml) was stirred overnight at room temperature, concentrated *in vacuo* and the residue azeotroped twice with toluene to give acetic 2-(3,4-diacetoxyphenyl)acetic mixed anhydride as a pale brown oil. This oil in water (100 ml) was heated under reflux for 15 minutes, allowed to cool, acidified to pH 2.5 with 5M hydrochloric acid and extracted with ethyl acetate (3 × 50 ml). The extracts were washed with brine (80 ml), dried over magnesium sulphate, filtered and evaporated to dryness to yield a brown solid which was crystallised from ethyl acetate-cyclohexane, 5.55 g, 94.5%, mp 101—102°C.

ν max ($CHCl_3$) 1770, 1715 and 1180 $cm^{-1}$.

δ ($CDCl_3$) 2.30 (6H, s, 2 × $CH_3CO_2$), 3.90 (2H, s, $CH_2CO_2$), 6.85—7.5 (3H, m, Ar), 10.05 (1H, bs, $CO_2H$).

This material was previously reported by Nagai, Chem. Ber. 1878, *11* 658 (Beilstein, Band X, 409), mp 89—90°C.

Example 1

2-(3,4-Diacetoxyphenyl)-2-sulphoacetyl chloride

2-(3,4-Diacetoxyphenyl)acetic acid (2.52 g, 10 mmole) in anhydrous dichloromethane (30 ml) and DMF (5 drops) was treated with oxalyl chloride (1 ml), stirred at room temperature for one hour and evaporated to dryness to afford 2-(3,4-diacetoxyphenyl)acetyl chloride as a pale yellow oil. This in anhydrous dichloromethane (30 ml) was cooled in an ice bath and treated dropwise with sulphur trioxide-dioxane complex in 1,2-dichloroethane (15 ml of a 1M solution, 15 mmole) and allowed to warm to room temperature overnight. This solution was then used in the next stage and was assumed to contain 10 mmoles of the title compound.

Example 2

Benzyl 6,β-[R,S-2-(3,4-diacetoxyphenyl)-2-sulphoacetamido]-6,α-methoxypenicillanate sodium salt

2-(3,4-Diacetoxyphenyl)-2-sulphoacetyl chloride (10 mmoles) in dichloromethane-1,2-dichloroethane was evaporated to dryness, the residue dissolved in THF (30 ml) cooled in an ice bath and treated with benzyl 6,β-amino-6,α-methoxypenicillanate (3.36 g, 10 mmole) and triethylamine (3.5 g, 35 mmole) in dichloromethane (30 ml). The mixture was allowed to warm to room temperature over two hours, concentrated *in vacuo* and the residue chromatographed on silica eluting with methanol in chloroform (0% grading to 10%) to afford the triethylammonium salt of the title compound, 1.98 g. This in water was passed through a column of 'Amberlite IR120 (Na)' ion exchange resin and the eluant freeze dried to give the title compound, 1.76 g, 26% yield.

ν max (KBr) 3680—2820, 1770, 1750, 1680, 1500, 1210, 1120, 1040 and 910 $cm^{-1}$.
δ [$(CD_3)_2SO$] 1.2—1.4 (6H, m, 2 × $2CH_3$), 2.25 (6H, s, 2 × $CH_3CO_2$), 3.40 (3H, s, $OCH_3$), 4.45, 4.57 (1H, 2 × s, 3H), 4.60, 4.88 (1H, 2 × s,CHCONH), 5.18 (2H, s, $OCH_2Ph$), 5.32, 5.38 (1H, 2 × s, 5H), 7.05—7.6 (8H, m, Ar), 9.52, 9.68 (1H, s × s,CONH).

### Example 3

Disodium 6,β-[R,S-2-(3,4-diacetoxyphenyl)-2-sulphoacetamido]-6,α-methoxypenicillanate

Benzyl 6,β-[R,S-2-(3,4-diacetoxyphenyl)-2-sulphoacetamido]-6,α-methoxypenicillanate sodium salt (0.53 g) in water (150 ml) was hydrogenated in the presence of 10% palladium on carbon (0.5 g). After one hour the mixture was filtered, the filtrate adjusted to pH 5.5 with dilute sodium hydroxide solution, fresh catalyst (0.5 g) added and hydrogenation continued for a further hour. The catalyst was filtered off, the filtrate again adjusted to pH 5.5, then freeze dried to yield the required material, 0.43 g, 89%.
ν max (KBr) 3750—2800, 1765, 1680, 1610, 1500, 1370, 1220 and 1080 $cm^{-1}$.
δ [$(CD_3)_2SO$] 1.35 (6H, bs, 2 × $2CH_3$), 2.25 (6H, bs, 2 × $CH_3CO_2$), 3.42 (3H, s, $OCH_3$), 4.05, 4.12 (1H, 2 × s, 3H), 4.50, 4.95 (1H, 2 × sCHCONH), 5.35, 5.42 (1H, 2 × s, 5H), 7.05—7.65 (3H, m, Ar), 9.45 (1H, bs, COHN).

### Example 4

Disodium 6,β-[R,S-2-(3,4-dihydroxyphenyl)-2-sulphoacetamido]-6,α-methoxypenicillanate

Disodium 6,β-[R,S-2-(3,4-diacetoxyphenyl)-2-sulphoacetamido]-6,α-methoxypenicillanate (0.032 g) in water (15 ml) was maintained at pH 10. by the addition of 0.05 M sodium hydroxide solution, for one hour, during which time the reaction mixture turned deep blue. A solution of sodium dithionite in water was added dropwise until the blue colour had been removed then the solution was chromatographed on "Sephadex G—25 fine' eluting with water. The fractions of eluant containing the product (detected by hplc on a $C_{18}$ υ-bondapak reverse phase column eluted with 8% methanol in 0.05 M ammonium acetate buffer, pH 4.5) were bulked and freeze dried to afford disodium 6,β-[R,S-2-(3,4-dihydroxyphenyl)-2-sulphoacetamido]-6,α-methoxypenicillanate, 0.012 g, 45% yield.
ν max (KBr) 3700—2800, 1765, 1675, 1610, 1570, 1410, 1340, 1230, 1205 and 1040 $cm^{-1}$.
δ [$(CD_3)_2SO$] 1.38 (6H, s, 2 × $2CH_3$), 3.37 (3H, s, $OCH_3$), 3.92, 3.98 (1H, s × s, 3H), 4.22, 4.50 (1H, 2 × s, CHCONH), 5.30, 5.34 (1H, 2 × s, 5H), 6.55—7.0 (3H, m, Ar), 9.2—9.35 (1H, s, CONH).

### Example 5

Benzyl 6,β-[R,S-2-(3,4-dihydroxyphenyl)-2-sulphoacetamido]-6,α-methoxypenicillanate sodium salt

Benzyl 6,β-[R,S-2-(3,4-diacetoxyphenyl)-2-sulphoacetamido]-6,α-methoxypenicillanate sodium salt (0.93 g, 1.4 mmole) in water (30 ml) was treated with sodium hydrogen carbonate (0.135 g, 2.8 mmole) and then maintained at pH 9.3 with 1M sodium hydroxide. The reaction was followed by hplc ($C_{18}$ 'μ-bondapak' reverse phase column eluted with 64% methanol in 0.05M ammonium acetate buffer, pH 4.5) and when no starting material remained the solution was adjusted to pH 6.3 with 1M hydrochloric acid, concentrated *in vacuo* to *ca* 5 ml and chromatographed on 'Sephadex G—25 fine' eluting with water. The fractions containing the product, detected by hplc ($C_{18}$ 'μ-bondapak' reverse phase column eluting with 56% methanol in 0.05 ammonium acetate buffer, pH 4.5) were bulked and freeze dried to yield the title compound, 0.19 g, 23.4%.
ν max (KBr) 3700—2700, 1770, 1745, 1680, 1500, 1210 and 1100 $cm^{-1}$.
δ [$(CD_3)_2SO$] 1.2—1.5 (6H, m, 2 × $2CH_3$), 3.38 (3H, s, $OCH_3$), 4.20, 4.45, 4.47, 4.55 (2h, 4 × s, 3H, CHCONH), 5.19 (2H, s, $OCH_2Ph$), 5.32, 5.35 (1H, 2 × s, 5H), 6.5—7.5 (8H, m, Ar), 8.5—8.8 (2H, m, 2 × OH), 9.43, 9.46 (1H, 2 × s, CONH).

### Example 6

Disodium 6,β-[R,S-2-(3,4-dihydroxyphenyl)-2-sulphoacetamido]-6,α-methoxypenicillanate

Benzyl 6,β-[R,S-2-(3,4-dihydroxyphenyl)-2-sulphoacetamido]-6,α-methoxypenicillanate sodium salt (0.2 g, 0.31 mmole) in water (15 ml) containing sodium hydrogen carbonate (0.025 g, 0.3 mmole) was hydrogenated in the presence of 10% palladium on carbon (0.15 g). The mixture was filtered through celite and the deep blue filtrate treated dropwise with 0.05 M aqueous sodium dithionite until colourless, then freeze dried to afford the title compound, 0.098 g, 61% yield. The spectral data for this compound agrees with that for the material prepared in Example 4.

### Example 7

Benzyl 6,β-[R-2-(3,4-diacetoxyphenyl)-2-sulphoacetamido]-6,α-methoxy-penicillanate N-methylmorpholine salt

R,S-2-(3,4-Diacetoxyphenyl)-2-sulphoacetyl chloride (75 mmole) in dichloromethane-1,2-dichloroethane (340 ml) was added dropwise to an ice cooled solution of benzyl 6,β-amino-6,α-methoxypenicillanate (25.2 g, 75 mmole) and N-methylmorpholine (29 ml) in dichloromethane (225 ml). The reaction mixture was allowed to warm to room temperature during one hour, washed with water, dried over magnesium sulphate, concentrated *in vacuo* and chromatographed on silica eluting with methanol in chloroform (0% grading to 5%). The first few fractions of the product, containing predominantly the S diastereoisomer, were discarded, then the bulk of the product was crystallised from 4-methylpentan-2-one

diisopropyl ether to give the R diastereoisomer, 4.25 g, 7.5% yield, mp 154—155°C, $[\alpha]_D^{20}$ 1% MeOH + 158.8.

ν max (KBr) 1775, 1750, 1690, 1500, 1110 and 1035 $cm^{-1}$.

δ $[(CD_3)_2SO]$ 1.25, 1.31 (6H, 2 × s, 2 × $2CH_3$), 2.24 (6H, s, 2 × $CH_3CO_2$), 2.75 (3H, s, morpholine $NCH_3$), 3.0—3.3 (4H, m, morpholine 3 and 5 $CH_2$), 3.42 (3H, s, $OCH_3$), 3.6—3.9 (4H, m, morpholine 2 and 6 $CH_2$), 4.48 (1H, s, 3H), 4.91 (1H, s, CHCONH), 5.19 (2H, s, $OCH_2Ph$), 5.34 (1H, s, 5H), 7.0—7.6 (8H, m, Ar), 9.55 (1h, s, CONH).

## Example 8

Disodium 6,β-[R-2-(3,4-diacetoxyphenyl)-2-sulphoacetamido]-6,α-methoxypenicillanate

Benzyl 6,β-[R-2-(3,4-diacetoxyphenyl)-2-sulphoacetamido]-6,α-methoxypenicillanate N-methylmorpholine salt (1.75 g) in water (80 ml) was hydrogenated in the presence of 10% palladium on carbon (0.40 g) for one hour. The mixture was filtered and the filtrate passed through an 'Amberlite IR120 (Na)' ion exchange column and the eluant freeze dried to give the title compound. 1.21 g, 86% yield.

ν max (KBr) 1765, 1680, 1610, 1215, 1110 and 1040 $cm^{-1}$.

δ $[(CD_3)_2SO]$ 1.30, 1.35 (6H, 2 × s, 2 × $2CH_3$), 2.24 (6H, s, 2 × $CH_3CO_2$), 3.40 (3H, s, $OCH_3$), 3.91 (1H, s, 3H), 4.81 (1H, s, CHCONH), 5.29 (1H, s, 5H), 7.05—7.45 (3H, m, Ar), 9.35 (1H, s, CONH).

## Example 9

Disodium 6,β-[R-2-(3,4-dihydroxyphenyl)-2-sulphoacetamido]-6,α-methoxypenicillanate

Disodium 6,β-[R-2-(3,4-diacetoxyphenyl)-2-sulphoacetamido]-6,α-methoxypenicillanate (1.28 g) in water (50 ml) was treated with citrus acetylesterase (75 units, 'Sigma' product No. A—7909) and maintained at pH 6.0 ± 0.1 with 0.2 M sodium hydroxide. When the uptake of sodium hydroxide has ceased the solution was concentrated *in vacuo* and chromatographed on 'Sephadex G—25 fine' eluting with water. The fractions containing the product were combined and freezing dried to give a quantitative yield of the title compound.

ν max 3700—2540, 1765, 1680, 1600, 1520, 1400 1240, 1200 and 1040 $cm^{-1}$.

δ $[(CD_3)_2SO]$ 1.38 (6H, s, 2 × $2CH_3$), 3.38 (3H, s, $OCH_3$), 3.92 (1H, s, 3H), 3.5—5.0 (2H, bs, 2 × OH), 4.48 (1H, s, CHCONH), 5.31 (1H, s, 5H, 6.5—6.9 (3H, m, Ar), 9.30 (1H, s, CONH).

Hplc analysis ($C_{18}$ 'μ-bondapak' reverse phase column eluted with 2.5% methanol in 0.1 M sodium phosphate buffer pH 4) showed <1% S diastereoisomer.

## Example 10

4-Nitrobenzyl 6,β-[2-(3,4-diacetoxyphenyl)-2-sulphoacetamido]-6,α-methoxypenicillanate triethylammonium salt

2-(3,4-Diacetoxyphenyl)-2-sulphoacetyl chloride (10 mmole) in 1,2-dichloroethane (40 ml) was added dropwise to 4-nitrobenzyl 6,β-amino-6,α-methoxypenicillanate (3.96 g, 10.4 mmole) and triethylamine (5 ml) in dichloromethane (20 ml). After 30 minutes, the solution was layered with water, stirred and adjusted to pH 6. The organic layer was collected, washed twice with water and evaporated to a foam which was chromatographed in silica eluting with chloroform followed by 5% methanol and 0.1% triethylamine in chloroform to give 4-nitrobenzyl 6,β-[S-2-(3,4-diacetoxyphenyl)2-sulphoacetamido]-6,α-methoxypenicillanate triethylammonium salt, 0.94 g, 14.8%, followed by 4-nitrobenzyl 6,β-[R-2-(3,4-diacetoxyphenyl)-2-sulphoacetamido]-6,α-methoxypenicillanate triethylammonium salt, 1.05 g, 16.6%.

δ $[(CD_3)_2SO]$ 1.1—1.7 (15H, m, $N(CH_2CH_3)_3$, 2 × $2CH_3$), 2.30 (6H, s, 2 × $OCOCH_3$), 3.13 (6H, q, J=7Hz, N $(CH_2CH_3)_3$), 3.60 (3H, s, $OCH_3$), 4.60 (1H, s, 3H), 5.13 (1H, s, CHCONH), 5.47 (2H, s, $CO_2CH_2$), 5.53 (1H, s, 5H), 7.1—8.5 (7H, n, Ar), 9.39 (1H—s, CONH).

## Example 11

Disodium 6,β-[R-2-(3,4-diacetoxyphenyl)-2-sulphoacetamido]-6,α-methoxypenicillanate

4-Nitrobenzyl 6,β-[R-2-(3,4-diacetoxyphenyl)-2-sulphoacetamido]-6,α-methoxypenicillanate triethylammonium salt (1.05 g) in water (30 ml) was hydrogenated in the presence of 10% palladium on carbon for 1.5 hours. The mixture was filtered and the filtrate, pH 3.5, passed through 'Amberlite 1R120 (Na)' resin and the eluant, pH 4.5, freeze dried to give the title compound, 0.57 g, 56.4%.

ν max (KBr) 1765, 1683, 1610, 1210 and 1041 $cm^{-1}$.

δ $[(CD_3)_2SO]$ 1.30, 1.35 (6H, 2 × s, 2 × $2CH_3$), 2.25 (6H, s, 2 × $OCOCH_3$), 3.40 (3H, s, $OCH_3$), 3.91 (1H, s, 3H), 4.81 (1H, s, CHCONH), 5.29 (1H, s, 5H), 7.0—7.5 (3H, m, Ar), 9.35 (1H, s, CONH).

Hplc analysis ($C_{18}$ 'μ-bondapak' reverse phase column eluted with 10% acetonitrile in 0.1 M sodium phosphate, pH 7.0) showed no detectable S diastereoisomer.

This product was identical with that in Example 8.

## Example 12

Benzyl 6,β-[R-2-(3,4-diacetoxyphenyl)-2-sulphoacetamido]-6,α-methylthio penicillanate N-methylmorpholine salt

2-(3,4-Diacetoxyphenyl)-2-sulphoacetyl chloride (20 mmole) in 1,2-dichloroethane (60 ml) was added dropwise to benzyl 6,β-amino-6,α-methylthiopenicillanate (7.04 g), 20 mmole) and N-methylmorpholine

(10 ml) in dichloromethane (25 ml) cooled in an ice bath. After the addition was complete the mixture was stirred for one hour, evaporated to dryness, dissolved in acetone, the insoluble N-methylmorpholinium chloride was discarded then the acetone was removed *in vacuo*. The residue was chromatographed on silica gel eluting with chloroform. Then with 5% methanol and 0.1% N-methylmorpholine in chloroform to give the S isomer, 2.87 g, 18.7%, a mixture of R and S isomers, 2.00 g, 13.0%, followed by the title compound, 3.28 g, 21.47%, $\delta[(CD_3)_2CO]$ 1.28, 1.40 (6H, 2 × s, 2 × $2CH_3$) 2.26 (6H, s, 2 × $COCH_3$), 2.41 (3H, s, $SCH_3$), 2.60 (3H, s, $NCH_3$), 2.7—3.2 and 3.5—4.0 (8H, m, morpholine), 4.46 (1H, s, 3H), 5.19 (2H, s, $O\underline{CH_2}Ph$), 5.27 (1H, s, $\underline{CH}CONH$), 5.40 (1H, s, 5H), 7.0—7.8 (8H, m, Ar), 9.12 (1H, s, CONH).

Example 13

Benzyl 6,β-[R-2-(3,4-diacetoxyphenyl)-2-sulphoacetamido]-6,α-methoxypenicillanate N-methylmorpholine salt

To a solution of benzyl 6,β-[R-(3,4-diacetoxyphenyl)-2-sulphoacetamido]-6,α-methylthiopenicillanate N-methylmorpholine salt (80 mgs, 1 mmol) in ethylacetate (30 mls) at 0°C was added a solution of mercuric acetate (0.35 gms, 1.1 mmol) in methanol (10 mls). After 20 minutes a 2% aqueous solution of N-methylmorpholinium sulphide (15 mls) was added. The black mercuric residues were removed by filtration through Celite and the organic phase separated. The aqueous phase was twice washed with ethyl acetate and the combined organic phases were dried over magnesium sulphate, filtered and evaporated *in vacuo* to afford a yellow foam. This foam was purified by silica gel column chromatography eluting with 10% methanol in 0.1% N-methylmorpholine containing chloroform to afford the title compound in 45% yield (0.34 gms) after crystallisation from 4-methylpentan-2-one.

The spectral data for this compound was in agreement with those listed in Example 7.

Example 14

Disodium 6,β-[R-2-(3,4-dihydroxyphenyl)-2-sulphoacetamido]-6,α-methoxypenicillanate

Disodium 6,β-[R-2-(3,4-diacetoxyphenyl)-2-sulphoacetamido]-6,α-methoxypenicillanate (100 mgs) was added in portions to an aqueous solution of the enzyme-Subtilisin Carlsberg (4 mgs, 4 mls) ('Sigma' Product No. P5380) maintained at pH 7.5 ± 0.2 with 0.05M sodium hydroxide. The reaction was monitored by hplc ($C_{18}$ 'μ-bondapak' reverse phase column eluting with 28% methanol in 0.05M ammonium acetate buffer, pH 4.5), and on completion it was acidified to pH 4.5 with dilute hydrochloric acid (0.1 M) and freeze dried to afford an off white solid. This solid was redissolved in water and chromatographed on 'XAD—2' resin eluting with water. The fractions containing the product were combined and freeze dried to afford the title compound in quantitative yield.

The spectral data was identical with that reported in Example 9.

Example 15

Disodium 6,β-[R-2-(3,4-dihydroxyphenyl)-2-sulphoacetamido]-6,α-methoxypenicillanate

Benzyl 6,β-[R-2-(3,4-diacetoxyphenyl)-2-sulphoacetamido]-6,α-methoxypenicillanate N-methylmorpholine salt (88 mgs) was added in portions to an aqueous solution of the enzyme Subtilisin Carlsberg ('Sigma' Product No. P 5380) (4mgs, 4 ml) maintained at pH 7.5 ± 0.2 with 0.05M sodium hydroxide. The reaction was monitored by hplc ($C_{18}$ 'μ-bondapak' reverse phase column eluting with 64% methanol in 0.05 M ammonium acetate buffer pH 4.5); on completion it was acidified to pH 4.5 with dilute hydrochloric acid (0.1 M) and freeze dried to afford a pale yellow solid (84 mgs). This material was purified by chromatography on 'XAD—2' resin eluting with water. The product containing fractions were bulked together and freeze dried to afford the title compound as an off white solid in 85% yield.

The spectral data for this material was in agreement with that reported in Example 9.

This material contained approximately 3% of the unwanted disodium 6,β-[S-2-(3,4-dihydroxyphenyl)-2-sulphoacetamido]-6,α-methoxypenicillanate.

Example 16

Disodium 6,β-[R-2-(3,4-dihydroxyphenyl)-2-sulphoacetamido]-6,α-methoxypenicillanate

Benzyl 6,β-[R-2-(3,4-diacetoxyphenyl)-2-sulphoacetamido]-6,α-methoxypenicillanate N-methylmorpholine salt (3.2 gms) was dissolved in water (40 mls). To this solution was added the enzyme Subtrilisin Alcalase (Novo Industries) immobilised on Mitsubishi WK103 resin (80 gms). The reaction mixture was mechanically stirred at 37° with the pH being maintained at pH 7.5 ± 0.2 by the addition of 2.5M sodium hydroxide. The reaction was monitored by hplc ($C_{18}$ 'μ-bondapak' reverse phase column eluting with 64% methanol in 0.05M ammonium acetate buffer pH 4.5) and on completion (*ca* 2.5 hours) the resin was filtered off and the solution acidified to pH 5.5 with IR 120 $H^+$ ion exchange resin. To the solution was added sufficient sodium chloride to make it 1 molar in sodium chloride. It was then bled onto the top of a 900 ml HP20 column which had been equilibrated with 1M sodium chloride solution at 5°C. The column was eluted with water and the fractions containing the product were bulked together and freeze dried to afford the title compound as a white solid in 67% yield (1.46 gms).

The spectral data for this material was in agreement with that reported in Example 9.

The material contained 1% of the unwanted disodium 6,β-[S-2-(3,4-dihydroxyphenyl)-2-sulphoacetamido]-6,α-methoxypenicillanate.

Example 17

Benzyl 6,β-[R-2-(3,4-diacetoxyphenyl)-2-sulphoacetamido]-6,α-methoxypenicillanate N-methylmorpholine salt

Benzyl 6,β-[S-2-(3,4-diacetoxyphenyl)-2-sulphoacetamido]-6,α-methoxypenicillanate N-methylmorpholine (7.5 gms, 10 mmol) was dissolved in water (75 mls) and adjusted to pH 8.6 with N-methylmorpholine. The solution was stirred at room temperature at this pH for 4 hours, the pH being maintained at 8.6 by the addition of N-methylmorpholine. The reaction mixture was then acidified to ph 5.5 by the addition of acetic anhydride. The aqueous solution was then washed with dichloromethane (3x). The combined organic solutions were dried over magnesium sulphate, filtered and evaporated *in vacuo* to afford a brown gum which was purified by chromatography on silica eluting with 10% methanol in chloroform. Then afforded the title compound in 33% yield (2.5 gms) after crystallisation from 4-methylpentan-2-one.

The spectral data for this compound were in agreement with those listed in Example 7.

Example 18

3,4-Diethyloxycarbonyloxyphenylacetic acid

3,4-Dihydroxyphenylacetic acid (1.68 g) was dissolved in water (50 ml) containing sodium bicarbonate (22 mM). After cooling to 5°, ethyl chloroformate (2.1 ml) was added dropwise, the pH being maintained at 7.5 by addition of sodium hydroxide solution. After stirring for thirty minutes consumption of base ceased and the pH was adjusted to 6.5 with dilute hydrochloric acid. The solution was extracted with ether (25 ml) then acidified to pH 2.0. This was extracted with ethyl acetate (2 × 25 ml) and the combined extracts washed with water (50 ml), dried over magnesium sulphate and evaporated. The residue was crystallised from toluene/cyclohexane to give the title compound as off-white crystals (2.56 g 82%); mp 78.5—80°; $\nu_{max}$ (Nujol) 3300, 1775 $cm^{-1}$; δ ($CDCl_3$) 1.37 (6H, t, J 7Hz, $\underline{CH_3}CH_2$), 3.66 (2H, s, $\underline{CH_2}CO_2H$), 4.33 (4H, q, J 7Hz, $CH_3\underline{CH_2}$), 7.29 (3H, bs, Ar), 9.90 (1H, bs, $CO_2H$).

Example 19

2-(3,4-Diethoxycarbonyloxyphenyl)-2-sulphoacetyl chloride

2-(3,4-Diethoxycarbonyloxyphenyl)-2-sulphoacetic acid (6.24 g, 20 mM) in dichloroethane (75 ml) was treated with oxalyl chloride (2.0 ml) DMF (2 drops). After stirring for one hour the solution was briefly degassed. $\nu_{max}$ ($C_2H_4Cl_3$) 1780 $cm^{-1}$.

To this solution was added a suspension of sulphur trioxide/dioxan complex in dichloroethane (20 ml, 20 mM) and the mixture stirred overnight at room temperature. This solution was taken as containing 20 mM of the title compound.

Example 20

Benzyl 6,β-[R-2-(3,4-diethoxycarbonyloxyphenyl)-2-sulphoacetamido]-6,α-methoxypenicillanate

Benzyl 6,β-amino-6,α-methoxypenicillanate (6.72 g 20 mM) in dichloromethane (60 ml) containing N-methylmorpholine (5.5 ml) was cooled in ice and treated with 2-(3,4-diethoxycarbonyloxyphenyl)-2-sulphoacetyl chloride (20 mM) in dichloroethane (70 ml). After stirring for thirty minutes at room temperature the solution was washed with dilute hydrochloric acid (75 ml), dried over magnesium sulphate and evaporated to a foam. This was chromatographed on silica eluting with 0—10% methanol in chloroform. The appropriate fractions were collected, evaporated and the title compound obtained by crystallisation from 2-methylpentan-4-one as its N-methylmorpholine salt. (1.46 g, 9%). $\nu_{max}$ (Nujol) 3250, 1785, 1765, 1750, 1695 $cm^{-1}$; δ [$(CD_3)_2SO$], 1.32 (12H, m, 2 × 2 $CH_3$, 2 × $\underline{CH_3}CH_2$), 2.75 (3H, s, N—$CH_3$), 3.22, 3.76 (9H, 2 × bs, morpholine H), 3.40 (3H, s, $OCH_3$), 4.22 (4H, q, J = 7Hz, 2 × $\underline{CH_2}CH_3$), 4.45 (1H, s, 3H), 4.86 (1H, s, CHCO), 5.16 (2H, s, $\underline{CH_2}Ph$), 5.30 (1H, s, 5H), 7.15—7.50 (8H, m, Ar), 9.50 (1H, s, NH).

Example 21

Disodium 6,β-[R-2-(3,4-diethoxycarbonyloxyphenyl)-2-sulphoacetamido]-6,α-methoxypenicillanate

Benzyl 6,β-[R-2-(3,4-diethoxycarbonyloxyphenyl)-2-sulphoacetamido]-6,α-methoxypenicillanate, N-methyl morpholine salt (0.3 g), in 20% aqueous tetrahydrofuran (50 ml) was hydrogenated with 10% palladium-charcoal catalyst (0.3 g) for thirty minutes at standard temperature and pressure. The solution was filtered and the solution passed down a column of Amberlyst IR 120 ($Na^+$) resin. The eluant was freeze-dried to yield the title compound (0.24 g 98%), $\nu_{max}$ (KBr), 3450, 1765, 1680, 1610 $cm^{-1}$; δ [$(CD_3)_2SO$], 1.25 (12H, m, 2 × 2$CH_3$, $\underline{CH_3}CH_2$), 3.40 (3H, s, $OCH_3$), 3.88 (1H, s, 3H), 4.25 (4H, q, J=8Hz, $CH_3\underline{CH_2}$), 4.84 (1H, s, CHCO), 5.27 (1H, s, 5H), 7.16—7.50 (3H, m, Ar), 9.33 (1H, s, NH).

Example 22

Disodium 6,β-[R-2-(3,4-dihydroxyphenyl)-2-sulphoacetamido]-6,α-methoxypenicillanate

Benzyl 6,β-[R-2-(3,4-diethoxycarbonyloxyphenyl)-2-sulphoacetamido]-6,α-methoxypenicillanate, N-methylmorpholine salt, (0.55 g) in water (10 ml) was added to a stirred suspension of Alcalase (Subtilisin Carlsberg) bound to Mitsubishu WK105 resin (10 g) in water (15 ml) at ph 7.5 and 37°C. After 4.5 hours the mixture was filtered and then Amberlyst IR 120 ($H^+$) resin added to the filtrate to give a pH of 3.5. This was

filtered and adjusted to pH 5.5 with dilute sodium hydroxide. The solution was freeze-dried to yield the title compound (0.32 g 91%).

Example 23

Phthalid-3-yl 6,β-[R-2-(3,4-diacetoxyphenyl)-2-sulphoacetamido]-6,α-methoxypenicillanate sodium salt

A mixture of disodium 6,β-[R-2-(3,4-diacetoxyphenyl)-2-sulphoacetamido]-6,α-methoxypenicillanate (0.6 g, 1 mmole) and 3-bromophthalide (0.426, 2 mmole) in dry dimethylformamide (5 ml) was stirred at room temperature for 18 hours then diluted with ether (150 ml). The ethereal solution was discarded and the residue chromatographed on silica eluting with 20% methanol in chloroform to yield 0.17 g, 23.8% of title compound. $\nu_{max}$ (KBr) 3450, 1770, 1685, 1505 and 1210 (br) $cm^{-1}$; δ [$(CD_3)_2CO$] 1.23, 1.45 (6H, 2 × s, 2 × $2CH_3$), 2.24 (6H, s, 2 × $CH_3CO$), 3.54 (3H, s, $OCH_3$), 4.62 (1H, s, 3H), 5.36 (1H, s, CHCONH), 5.51 (1H, s, 5H), 7.13—8.25 (8H, m, Ar, phthalidyl), 9.21 (1H, s, CONH).

Example 24

Acetoxymethyl 6,β-[R-2-(3,4-diacetoxyphenyl)-2-sulphoacetamido]-6,α-methoxypenicillanate

Disodium 6,β-[R-2-(3,4-diacetoxyphenyl)-2-sulphoacetamido]-6,α-methoxypenicillanate (0.42 g) in DMF (2 ml) was treated with acetoxymethyl bromide (130 μl) and sodium bicarbonate (0.1 g). Stirred for two and a half hours- then the solvent was evaporated. The residue was chromatographed on silica eluting with 10% methanol in chloroform. The appropriate fractions were evaporated, taken up in water, filtered and freeze-dried to yield the title compound (0.115 g 25%); $\nu_{max}$ (KBr), 1760 ,1675 $cm^{\pm1}$; δ [$(CD_3)_3SO$], 1.26, 1.32 (6H, 2 × s, 2 × $2CH_3$), 2.03 (3H, s, $CH_2$.O.CO.$CH_3$), 2.22 (6H, s, 0.CO.$CH_3$) 3.40 (3H, s, $OCH_3$), 4.46 (1H, s, 3H), 4.81 (1H, s, CHCO), 5.30 (1H, s, 5H), 5.72 (2H, s, $CH_2$O.CO.$CH_3$), 7.15—7.45 (3H, m, Ar) 9.47 (1H, s, NH).

Example 25

Pivaloyloxymethyl 6,β-[3,4-diacetoxyphenyl)-2-sulphoacetamido]-6,α-methoxypenicillanate sodium salt

A mixture of disodium 6,β-[R-2-(3,4-diacetoxyphenyl)-2-sulphoacetamido]-6,α-methoxypenicillanate (0.6 gm, 1 mmole) and bromomethylpivaloate (0.39 gm, 2 mmole) in dry dimethylformamido (1 ml) was stirred at room temperature for 4 hours whilst the pH was maintained at pH 7 by the addition of sodium bicarbonate (0.084 gm, 1 mmole). The solvent was removed *in vacuo* and the residue chromatographed on silica eluting with 0.1% N-methylmorpholine salt of the title compound as a clear glass. This glass was redissolved in water converted to the sodium salt by treatment with IR 120 $Na^+$ ion exchange resin and freeze dried to yield 0.25 gm, 36% of the title compound. ν (KBr) 3460, 1765, 1504, 1372, 1210, 1160, 1045, 990 $cm^{-1}$; δ [$(CD_3)_2SO$] 1.12 (9H, s, $C(CN_3)_3$), 1.29, 1.35 (6H, 2 × s, 2 × $2CH_3$), 2.23 (6H, s, 2 × $CH_3CO$), 3.40 (3H, s, $OCH_3$), 4.48 (1H, s, 3H), 4.81 (1H, s, CHCONH), 5.31 (1H, s, 5H), 5.71, 5.85 (2H, q, J=6Hz, $OCH_2O$), 7.00—7.31 (3H, m, Ar) 9.50 (1H, s, CHCONH).

Example 26

Phthalidyl 6,β-[R-2-(3,4-dihydroxyphenyl)-2-sulphoacetamido]-6,α-methoxypenicillanate

Disodium 6,β-[R-2-(3,4-dihydroxyphenyl)-2-sulphoacetamido]-6,α-methoxypenicillanate (297 mg) in dimethylformamide (4 ml) was treated with 3-bromophthalide (140 mg). After stirring for 3½ hours at room temperature the solvent was evaporated under vacuum and the residue purified by chromatography on silica, eluting with 20% methanol in chloroform. The purified material was taken up in water, filtered and freeze-dried (182 mg, 51%); $\nu_{max}$ (KBr), 1740, 1665, 1610 $cm^{-1}$; δ [$(CD_3)_2SO$] 1.37 (6H, bs, 2 × $2CH_3$), 3.38 (3H, s, $OCH_3$), 4.43 (1H, s, 3H), 4.60 (1H, s, CHCO), 5.32 (1H, s, 5H), 6.55—8.05 (8H, m, Ar, Phthalide CH), 8.50, 8.63 (2H, 2 × s, OH), 9.47 (1H, s, NH).

BIOLOGICAL DATA

The antibacterial activities of a number of compounds of the present invention

MIC (µg/ml)

| Compound of Example No. | 8 | | 3 | | 9 | | 4 | |
|---|---|---|---|---|---|---|---|---|
| Organism | Agar | Broth | Agar | Broth | Agar | Broth | Agar | Broth |
| E. coli ESS | 0.2 | | 0.2 | | 0.1 | | 0.5 | |
| E. coli JT 4 | 2.5 | | 2.5 | | 2.5 | | 5.0 | |
| E. coli JT 425 | 1.0 | | 2.5 | | 1.0 | | 5.0 | |
| E. coli NCTC 10418 | 2.5 | 5.0 | 2.5 | 5.0 | 2.5 | | 5.0 | |
| Ps. aeruginosa NCTC 10662 | 2.5 | 1.0 | 2.5 | 5.0 | 2.5 | | 2.5 | |
| Ps. aeruginosa NCTC 10662 $10^{-2}$ | 0.5 | 0.5 | 2.5 | 5.0 | 0.5 | | 5.0 | |
| Ps. aeruginosa Dalgleish $10^{-2}$ | 0.5 | | 2.5 | | 0.5 | | 2.5 | |
| S. marcescens US 32 | 25.0 | | 25.0 | | 25.0 | | 25.0 | |
| K. aerogenes A | 5.0 | 10.0 | 10.0 | 25.0 | 5.0 | | 5.0 | |
| E. cloacae N1 | 25.0 | | 50.0 | | 50.0 | | 50.0 | |
| P. mirabilis C 977 | 10.0 | | 25.0 | | 25.0 | | 25.0 | |

**Claims**

1. A compound of formula (I) or a pharmaceutically acceptable salt or *in vivo* hydrolysable ester thereof:

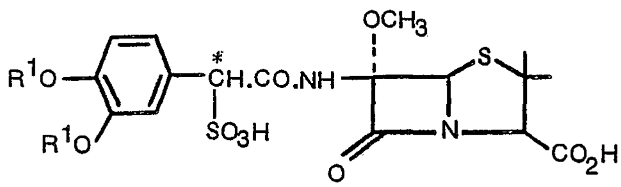

(I)

wherein $R^1$ is hydrogen or acetyl.

2. A compound as claimed in claim 1 wherein the carbon marked * in formula (I) is in the R configuration.

3. 6,β-[R,S-2-(3,4-dihydroxyphenyl)-2-sulphoacetamido]6,α-methoxypenicillanic acid or a pharmaceutically acceptable salt or *in vivo* hydrolysable ester thereof.

4. 6,β-[R-2-(3,4-dihydroxyphenyl)-2-sulphoacetamido]6,α-methoxypenicillanic acid or a pharmaceutically acceptable salt or *in vivo* hydrolysable ester thereof.

5. Disodium 6,β-[R-2-(3,4-dihydroxyphenyl)-2-sulphoacetamido]-6,α-methoxypenicillanate.

6. A process for the preparation of a compound as claimed in claim 1 which process comprises (a) reacting a compound of formula (II):

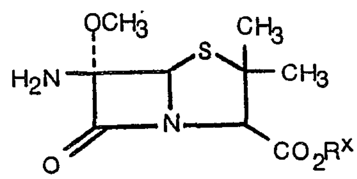

(II)

wherein the amino group is optionally substituted with a group which permits acylation to take place, and wherein $R^x$ is hydrogen or a carboxyl-blocking group, with an N-acylating derivative of an acid of formula (III):

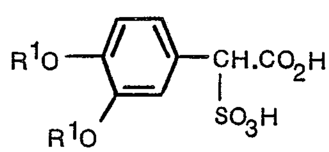

(III)

wherein $R^1$ is defined with respect to formula (I) above; or
(b) reacting a compound of formula (IV):

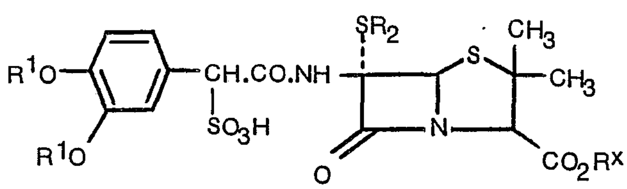

(IV)

wherein $R^1$ and $R^x$ are as defined above and $R^2$ is $C_1$ to $C_6$ alkyl, aryl or benzyl; with methanol in the presence of a metal ion; or
(c) treating a compound of formula (V):

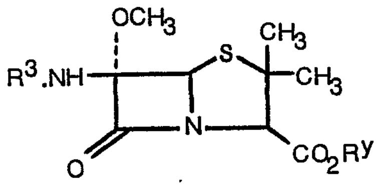

(V)

wherein $R^y$ is a carboxyl-blocking group, and $R^3$ is an acyl group, with an agent forming an imino halide; treating the imino halide with a compound to introduce a group $QR_f$ on the imino carbon atom, wherein Q is oxygen, sulphur or nitrogen and $R_f$ is an alkyl group of from 5 to 14 carbon atoms, to form an iminoether, iminothioether, or amidine (when Q is O, S, or N respectively); reacting with an N-acylating derivative of an acid of formula (III); treating with water; and after any of processes a), b) or c), if necessary, carrying out one or more of the following steps:
i) removing any carboxyl-blocking group;
ii) removing any substituent on the amide group;
iii) converting the product into a salt or *in vivo* hydrolysable ester thereof.

7. A pharmaceutical composition comprising a compound as claimed in any one of claim 1 to 5 together with a pharmaceutical carrier or excipient.

8. A composition as claimed in claim 7 which further comprises a β-lactamase inhibitor.

9. A composition as claimed in claim 8 wherein the β-lactamse inhibitor is a compound of formula (VI) or a pharmaceutically acceptable salt or ester thereof:

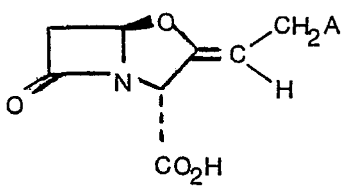

(VI)

wherein A is hydroxyl, substitued hydroxyl, thiol, substituted thiol, amino, mono- or di-hydrocarbyl-substituted amino, or mono- or di-acylamino.

**Revendications**

1. Composé de formule (I) ou un sel acceptable du point de vue pharmaceutique ou un ester hydrolysable in-vivo de celui-ci,

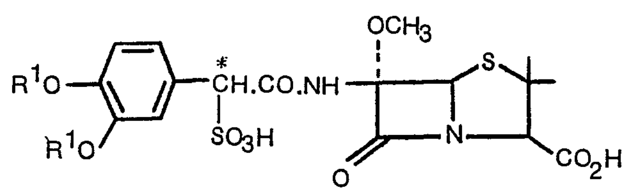

(I)

dans laquelle $R^1$ est un atome d'hydrogène ou un groupe acétyle.

2. Composé suivant la revendication 1, caractérisé en ce que l'atome de carbone marqué * dans la formule (I) est en configuration R.

3. Acide 6,β-[R,S-2-(3,4-dihydroxyphényl)-2-sulfoacétamido]-6,α-méthoxypénicillanique ou un sel acceptable du point de vue pharmaceutique ou un ester hydrolysable in-vivo de celui-ci.

4. Acides 6,β-[R-2-(3,4-dihydroxyphényl)-2-sulfoacétamido]-6,α-méthoxypénicillanique ou un sel acceptable du point de vue pharmaceutique ou un ester hydrolysable in-vivo de celui-ci.

5. 6,β-[R-2-(3,4-dihydroxyphényl)-2-sulfoacétamido]-6,α-méthoxypénicillanic disodique.

6. Procédé de préparation d'un composé suivant la revendication 1, caractérisé en ce qu'il comprend:
a) la réaction d'un composé de formule (II) ci-après:

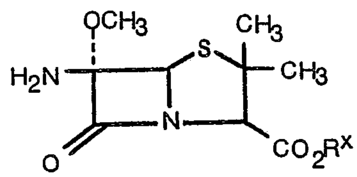

(II)

dans laquelle le groupe amino est éventuellement substitué avec un groupe qui permet la réalisation d'une acylation, et où $R^x$ est un atome d'hydrogène ou un groupe bloquant le radical carboxyle, avec un dérivé N-acylant d'un acide de formule (III) ci-après:

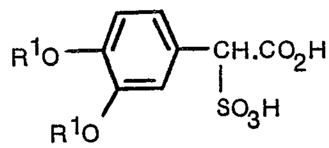

(III)

dans laquelle $R^1$ est tel que défini à propos de la formule (I) ci-dessus; ou
b) la réaction d'un composé de formule (IV) ci-après:

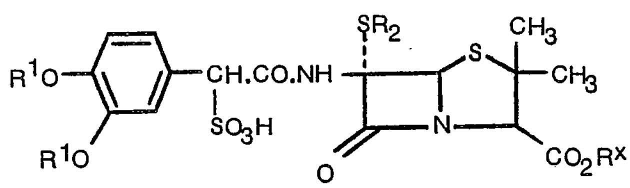

(IV)

dans laquelle $R^1$ et $R^x$ sont tels que définis ci-dessus et $R^2$ est un groupe alcoyle en $C_{1-6}$, aryle ou benzyle; avec du méthanol en présence d'un ion métallique; ou
c) le traitement d'un composé de formule (V) ci-après:

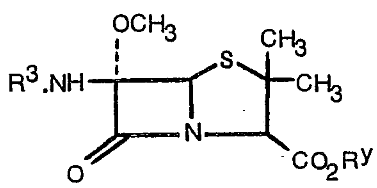

(V)

dans laquelle $R^y$ est un groupe bloquant la fonction carboxyle et $R^3$ est un groupe acyle, avec un agent formant um iminohalogénure; le traitement de l'iminohalogénure avec un composé pour introduire un groupe $QR_f$ sur l'atome de carbone du radical imino, où Q est un atome d'oxygène, de soufre ou d'azote et $R_f$ est un groupe alcoyle contenant 5 à 14 atomes de carbone, pour former un iminoéther, un iminothioéther ou une amidine (lorsque Q est O, S ou N respectivement); la réaction avec un dérivé N-acylant d'un acide de formule (III); le traitement avec de l'eau; et après l'un quelconque des procédés a), b) ou c), si cela est nécessaire, la réalisation d'une ou de plusieurs des étapes suivantes:
i) l'élimination d'un quelconque groupe bloquant la fonction carboxyle;
ii) l'élimination d'un quelconque substituant sur le groupe amide;
iii) la conversion du produit en un sel ou en un ester hydrolysable in-vivo de celui-ci.

7. Composition pharmaceutique caractérisée en ce qu'elle comprend un composé suivant l'une quelconque des revendications 1 à 5, avec un excipient ou un support pharmaceutique.

8. Composition suivant la revendication 7, caractérisée ce qu'elle comprend de plus un inhibiteur de β-lactamase.

9. Composition suivant la revendication 8, caractérisée en ce que l'inhibiteur de β-lactamase est un composé représenté par la formula (VI) ci-après: un ester ou un sel acceptable du point de vue pharmaceutique de celui-ci

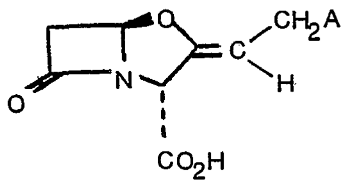

(VI)

dans laquelle A est un groupe hydroxy, hydroxy substitué, thiol, thiol substitué, amino, amino mono- ou di-hydrocarbyl substitué, ou mono-ou di-acylamino.

**Patentanspruche**

1. Eine Verbindung der Formel (I) oder ein pharmakologisch zulässiges Salz oder eine in vivo hydrolysierbarer Ester derselben:

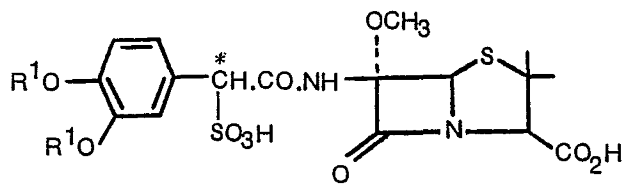

(I)

in welcher $R^1$ Wasserstoff oder Acetyl ist.

2. Eine Verbindung wie in Anspruch 1 beansprucht, in welcher sich das mit * markierte Kohlenstoffatom in Formel (I) in der R-Konfiguration befindet.

3. 6,β-[R,S-2-(3,4-Dihydroxyphenyl)-2-sulfoacetamido]-6,α-methoxypenicillansäure oder ein pharmakologisch zulässiges Salz oder ein in vivo hydrolysierbarer Ester derselben.

4. 6,β-[R-2-(3,4-Dihydroxyphenyl)-2-sulfoacetamido]-6,α-methoxypenicillansäure oder ein pharmakologishch zulässiges Salz oder ein in vivo hydrolysierbarer Ester derselben.

5. Di-natrium-6,β-[R-2-(3,4-dihydroxyphenyl)-2-sulfoacetamido]-6,α-methoxypenicillansäure salz.

6. Ein Verfahren zur Herstellung einer Verbindung wie in Anspruch 1 beansprucht, welches Verfahren a) das Umsetzen einer Verbindung der Formel (II):

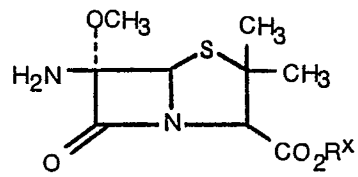

(II)

in welcher die Aminogruppe gegebenenfalls mit einer die Durchführung einer Acylierung erlaubenden Gruppe substituiert ist und in welcher $R^x$ Wasserstoff oder eine die Carboxylgruppe blockierende Gruppe bedeutet, mit einem N-acylierenden Derivat einer Säure der Formel (III):

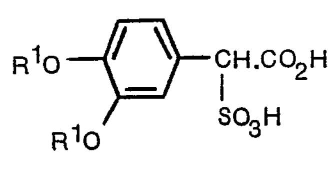

(III)

in welcher $R^1$ wie in Bezug auf Formel (I) definiert ist; oder

(b) das Umsetzen einer Verbindung der Formel (IV):

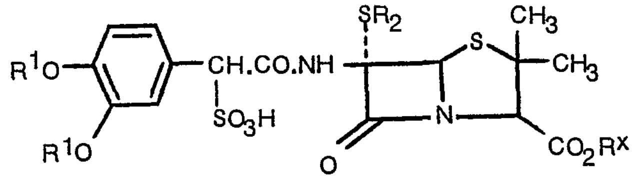

(IV)

in welcher $R^1$ und $R^x$ wie vorstehend definiert sind und $R^2$ $C_{1-6}$-Alkyl, Aryl oder benzyl bedeutet, mit Methanol in Anwesenheit eines Metallions; oder

(c) das Behandeln einer Verbindung der Formel (V):

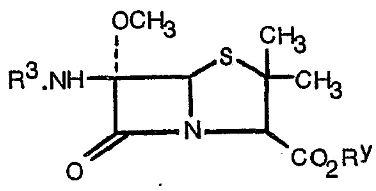

(V)

in welcher $R^y$ eine die Carboxylgruppe blockierende Gruppe und $R^3$ eine Acylgruppe ist, mit einem ein Iminohalogenid bildenden Mittel; das Behandeln des gebildeten Iminohalogenids mit einer Verbindung zwecks Einführung einer Gruppe $QR_f$ an das Iminokohlenstoffatom, wobei Q Sauerstoff, Schwefel oder Stickstoff bedeutet und $R_f$ eine Alkylgruppe mit 5 bis 14 Kohlenstoffatomen darstellt, unter Bildung eines Iminoäthers, Iminothioäthers oder Amidins (wenn Q Sauerstoff bwz. Schwefel bzw. Stickstoff bedeutet); Umsetzen mit einem N-acylierenden Derivat einer Säure der Formel (III); Behandeln mit Wasser; und im Anschluß an irgendeines der Vorstehenden Verfahren (a), (b) oder (c) erforderlichenfalls die Durchführung einer oder mehrerer der nachstehenden Verfahrensstufen umfaßt:

i) Entfernen irgendeiner eine Carboxylgruppe blockierenden Gruppe;

ii) Entferen irgendeines an der Amidgruppe befindlichen Substituenten;

iii) Umwandeln des Reaktionsproduktes in ein Salz oder einen in vivo hydrolysierbaren Ester desselben.

7. Eine pharmazeutische Zusammensetzung, enthaltend eine Verbindung wie in irgendeinem der Ansprüche 1 bis 5 beansprucht, zusammen mit einem pharmazeutischen Trägermaterial oder Exzipienten.

8. Eine Zusammensetzung wie in Anspruch 7 beansprucht, welche zusätzlich einen β-Lactamase-Inhibitor enthält.

9. Eine Zusammensetzung wie-in Anspruch 8 beansprucht, in welcher der β-Lactamase-Inhibitor eine Verbindung der Formel (VI) oder ein pharmakologisch zulässiges Salz oder ein Ester derselben ist:

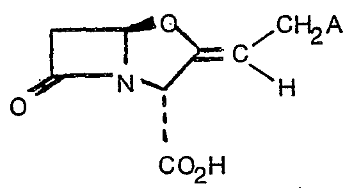

(VI)

in welcher A Hydroxyl, substituiertes Hydroxyl, Thiol, substituiertes Thiol, Amino-, Mono- oder Dihydrocarbylsubstituiertes Amino- oder Mono- oder Diacylamino bedeutet.